# EUROPÄISCHE PATENTANMELDUNG

(11) **EP 4 660 129 A2**
(43) Veröffentlichungstag der Anmeldung: **10.12.2025**
(21) Anmeldenummer: 25211371.7
(22) Anmeldetag: 22.06.2018
(51) Int. Cl.: B67C 7/00

(54) **ANLAGE UND VERFAHREN ZUM HERSTELLEN VON GETRÄNKEBEHÄLTNISSEN MIT STERILISATION DER BLASFORMMASCHINE**

(30) Priorität: 03.07.2017 DE 102017114766
(62) Teilanmeldung aus: 18743687.8
(71) Anmelder: Krones AG, 93073 Neutraubling (DE)
(72) Erfinder: MÜLLER, Holger, 93073 Neutraubling (DE); GELTINGER, Florian, 93073 Neutraubling (DE); SÖLLNER, Jürgen, 93073 Neutraubling (DE)
(74) Vertreter: Bittner, Bernhard

(57) **Zusammenfassung**

Vorrichtung zum Herstellen von Behältnissen und insbesondere von Getränkebehältnissen mit einer Erwärmungseinrichtung (2), welche dazu geeignet und bestimmt ist, Kunststoffvorformlinge zu erwärmen, mit wenigstens einer ersten Transporteinrichtung (12), welche dieses Kunststoffvorformlinge (10) entlang eines vorgegebenen Transportpfads transportiert, mit einer Sterilisationseinrichtung (4), welche dazu geeignet und bestimmt ist, die Kunststoffvorformlinge (10) zu sterilisieren und mit einer in einer Transportrichtung der Kunststoffvorformlinge (10) nach der Sterilisationseinrichtung (4) angeordneten Umformungseinrichtung (6), welche dazu geeignet und bestimmt ist, die Kunststoffvorformlinge zu Kunststoffbehältnissen durch Beaufschlagung mit einem fließfähigen Medium umzuformen, wobei diese Umformungseinrichtung (6) einen Transportraum (62) aufweist, innerhalb dessen die Kunststoffvorformlinge (10) transportiert werden. Erfindungsgemäß weist die Vorrichtung (1) eine Beaufschlagungseinrichtung (64) auf, welche diesen Transportraum (62) wenigstens zeitweise mit einem fließfähigen Sterilisationsmedium beaufschlagt.

## Beschreibung

Die vorliegende Erfindung bezieht sich auf eine Vorrichtung und ein Verfahren zum Herstellen von Getränkebehältnissen. Derartige Verfahren sind aus dem Stand der Technik seit Langem bekannt. Bei einem bekannten Verfahren werden zunächst Kunststoffvorformlinge erwärmt, anschließend sterilisiert, anschließend mittels einer Umformungseinrichtung, wie beispielsweise einer Streckblasmaschine, zu Kunststoffbehältnissen umgeformt und schließlich bevorzugt in diesem umgeformten Zustand mit einer Flüssigkeit und insbesondere einem Getränk befüllt. Je nach dem abzufüllenden Getränk sind dabei unterschiedliche Sterilisations- oder Reinheitsgrade bekannt. So sind aus dem Stand der Technik in jüngerer Zeit auch sterile Blasformmaschinen bekannt geworden, welche ein Herstellen von Kunststoffflaschen unter sterilen bzw. Reinraumbedingungen ermöglichen. Derartige Maschinen werden nach allen Kriterien der Aseptik und Hygiene hinsichtlich Verfahrenstechnik und -konstruktion entwickelt.

Allerdings existieren auch Produkte, welche unter geringeren hygienischen Anforderungen abgefüllt werden können. Für diese Produkte wurden Herstellungsanlagen entwickelt, welche im Gegensatz zum vollständig aseptisch ausgeprägten Transportpfad nach einer Behälterentkeimung bzw. Preform-Entkeimung bis hin zum Füllprozess an Stelle einer sterilen Blasmaschine mit einer nicht sterilen oder herkömmlichen Blasmaschine auskommen. In solchen Fällen ist es aus dem internen Stand der Technik der Anmelderin bekannt, dass der Blasmaschine hier lediglich konditionierte Luft, beispielsweise gefilterte und getrocknete Luft, zugeführt wird, um potentiell unsaubere Luft von dem Modul fernzuhalten und auch um Wasserkondensation in dem Blasmodul bzw. der Blasformmaschine zu vermeiden. Die unterschiedlichen Maschinen sind dabei üblicherweise passend für jede Anwendung konzipiert. Allerdings besteht teilweise ein Bedarf an einem Maschinentyp, der nicht notwendigerweise den sehr aufwändigen kompletten sterilen Transportweg bietet, auf der anderen Seite jedoch auch einen höheren Reinheitsgrad zur Verfügung stellt als vollständig nicht aseptische Anlagen.

Der vorliegenden Erfindung liegt daher die Aufgabe zugrunde, eine Vorrichtung und ein Verfahren zur Verfügung zu stellen, welche einerseits noch einen vertretbaren Reinheitsgrad der hergestellten Behältnisse bieten, andererseits jedoch nicht notwendigerweise vollständig steril gehalten sind und auf diese Weise günstiger in der Herstellung und/oder im Betrieb sind. Diese Aufgaben werden erfindungsgemäß durch die Gegenstände der unabhängigen Patentansprüche erreicht. Vorteilhafte Ausführungsformen und Weiterbildungen sind Gegenstand der Unteransprüche.

Eine erfindungsgemäße Vorrichtung zum Herstellen von Behältnissen und insbesondere von Getränkebehältnissen weist eine Erwärmungseinrichtung auf, welche dazu geeignet und bestimmt ist, Kunststoffvorformlinge zu erwärmen. Weiterhin weist die Vorrichtung eine erste Transporteinrichtung auf, welche diese Kunststoffvorformlinge entlang eines vorgegebenen Transportpfads transportiert sowie eine Sterilisationseinrichtung, welche dazu geeignet und bestimmt ist, die Kunststoffvorformlinge zu sterilisieren. Weiterhin weist die Vorrichtung eine in einer Transportrichtung der Kunststoffvorformlinge nach der Sterilisationseinrichtung angeordnete Umformungseinrichtung auf, welche dazu geeignet und bestimmt ist, die Kunststoffvorformlinge zu Kunststoffbehältnissen durch Beaufschlagung mit einem fließfähigen Medium umzuformen. Dabei weist diese Umformungseinrichtung einen Transportraum auf, innerhalb dessen die Kunststoffvorformlinge transportiert werden.

Erfindungsgemäß weist die Vorrichtung eine Beaufschlagungseinrichtung auf, welche diesen Transportraum wenigstens zeitweise und bevorzugt permanent mit einem fließfähigen Sterilisationsmedium beaufschlagt. Bevorzugt beaufschlagt die Beaufschlagungseinrichtung den Transportraum auch während der Produktion. Bevorzugt handelt es sich bei dieser Beaufschlagung um ein Begasen.

Bei der genannten Umformungseinrichtung handelt es sich insbesondere um eine Blasformmaschine und besonders bevorzugt um eine Streckblasmaschine. Diese Umformungseinrichtung weist dabei bevorzugt Blasformen auf, welche an den einzelnen Umformungsstationen angeordnet sind. Diese Blasformen sind dabei bevorzugt wenigstens mittelbar an Blasformträgern angeordnet und können mittels dieser Blasformträger geöffnet und geschlossen werden. Weiterhin weisen die einzelnen Umformungsstationen bevorzugt jeweils stangenartige Körper auf, welche in die Kunststoffvorformlinge einführbar sind, um diese in deren Längsrichtung zu dehnen. Bevorzugt weist die Umformungseinrichtung einen drehbaren Träger, das sogenannte Blasrad auf, an dem eine Vielzahl von Umformungsstationen angeordnet ist.

Dabei ist es möglich, dass die besagte Umformungseinrichtung einen Reinraum aufweist, innerhalb dessen die Kunststoffvorformlinge transportiert und umgeformt werden. Bevorzugt weist jedoch diese Maschine keinen derartigen Reinraum auf und ist damit bevorzugt nicht aseptisch ausgeführt.

Bevorzugt weist jedoch diese Umformungseinrichtung ein Gehäuse auf, innerhalb dessen die Kunststoffvorformlinge transportiert und/oder umgeformt werden. Dieses Gehäuse kann dabei im Wesentlichen zwei Öffnungen aufweisen, wobei die Kunststoffvorformlinge durch eine dieser Öffnungen in die Umformungseinrichtung transportiert werden und durch eine zweite Öffnung wieder als umgeformte Flaschen aus der Umformungseinrichtung heraus transportiert werden.

Die Erfindung schlägt vor, innerhalb der Umformungseinrichtung eine gewisse Sterilisationsmittelatmosphäre zu schaffen, um auf diese Weise den Reinheitsgrad in der Umformungseinrichtung zu erhöhen. Bevorzugt kann dabei eine Beaufschlagung von Bauteilen der Umformungseinrichtung stattfinden, etwa der Blasformen, deren Träger oder dergleichen. Daneben ist es jedoch bevorzugt, dass innerhalb des Raumes lediglich eine Sterilisationsmittel - Atmosphäre geschaffen wird.

Bei einer bevorzugten Ausführungsform weist die Vorrichtung eine Beaufschlagungseinrichtung auf, welche die Kunststoffvorformlinge zu deren Umformung und/oder Expansion mit einem fließfähigen Medium beaufschlagt. Bei diesem fließfähigen Medium kann es sich insbesondere um Luft, insbesondere um Blasluft oder Sterilluft handeln, welche in die Kunststoffvorformlinge geblasen wird, um diese zu expandieren. Es wäre jedoch auch möglich, dass es sich bei dem fließfähigen Medium um eine Flüssigkeit handelt, insbesondere um das abzufüllende Getränk, welches in die Kunststoffvorformlinge eingefüllt wird, sodass die Kunststoffvorformlinge durch das abzufüllende Produkt auch mit expandiert werden. In diesem Falle wäre die Umformungseinrichtung gleichzeitig Umformungseinrichtung und auch Füllmaschine.

Bei dem fließfähigen Sterilisationsmedium, mit welchem der Transportraum bzw. der Umformungsraum beaufschlagt wird, kann es sich insbesondere aber nicht ausschließlich um Wasserstoffperoxid (H₂O₂) oder Peressigsäure handeln.

Bevorzugt handelt es sich bei der Erwärmungseinrichtung, welche der Umformungseinrichtung vorgeschaltet ist, um einen Durchgangsofen. Innerhalb dieses Durchgangsofens werden die Kunststoffvorformlinge bevorzugt erwärmt, was insbesondere durch Infrarotheizelemente erfolgen kann. Es wäre jedoch auch möglich, dass es sich bei dem Ofen um einen Mikrowellenofen handelt, der die Kunststoffvorformlinge durch Beaufschlagung mit Mikrowellen erwärmt. Auch wäre es möglich, dass es sich bei der Erwärmungseinrichtung direkt um eine Spritzgießmaschine handelt, das heißt die Kunststoffvorformlinge direkt aus einer Spritzgießmaschine stammen und daher noch eine hohe Eigentemperatur aufweisen. In diesem Falle wäre die Spritzgießmaschine auch gleichzeitig die besagte Erwärmungseinrichtung.

Bei einer weiteren vorteilhaften Ausführungsform ist die Sterilisationseinrichtung dazu geeignet und bestimmt, zumindest auch die Innenräume der Kunststoffvorformlinge mit einem Sterilisationsmittel und insbesondere einem fließfähigen Sterilisationsmedium zu beaufschlagen. Zusätzlich oder alternativ wäre es jedoch auch denkbar, dass diese Sterilisationseinrichtung Strahlungseinrichtungen aufweisen, welche zumindest Abschnitte der Wandungen der Kunststoffvorformlinge mit Strahlung zum Zwecke der Sterilisation beaufschlagen. Bei diesen Strahlungen kann es sich beispielsweise um Elektronenstrahlung handeln.

Vorzugsweise beaufschlagt dabei diese Strahlungseinrichtung insbesondere auch eine Innenoberfläche der Kunststoffvorformlinge.

Bei einer weiteren vorteilhaften Ausführungsform steht die Sterilisationseinrichtung mit der Umformungseinrichtung derart in Strömungsverbindung, dass ein Sterilisationsmittel von der Sterilisationseinrichtung zu der Umformungseinrichtung gelangen kann. So könnten beispielsweise Gehäuseteile der Sterilisationseinrichtung und der Umformungseinrichtung mittels einer Öffnung miteinander verbunden werden, wobei die Kunststoffvorformlinge durch diese Öffnung hindurch transportierbar sind.

Bei einer weiteren vorteilhaften Ausführungsform ist daher die Konzentration des Sterilisationsmediums innerhalb des Sterilisationsmoduls bzw. innerhalb der Sterilisationseinrichtung höher als in der Umformungseinrichtung. Das Sterilisationsmedium kann insbesondere durch eine Schleuse von der Sterilisationseinrichtung zu der Umformungseinrichtung gelangen. Diese Schleuse stellt damit hier die Beaufschlagungseinrichtung dar, welche dazu geeignet und bestimmt ist, den Transportraum der Umformungseinrichtung mit dem Sterilisationsmedium zu beaufschlagen.

Vorteilhaft weist daher auch die Sterilisationseinrichtung ein Gehäuse bzw. einen Gehäuseraum auf, innerhalb dessen die Sterilisation der Kunststoffvorformlinge erfolgt. Es ist weiterhin auch bevorzugt eine Beaufschlagungseinrichtung vorgesehen, welche die Sterilisationseinrichtung und insbesondere auch diesen Sterilisationsraum mit einem Sterilisationsmedium beaufschlagt.

Es wird daher vorgeschlagen, dass die Umformungseinrichtung unter Nutzung geringer Sterilisationsmittelkonzentration einer sterilisierenden Atmosphäre ausgesetzt wird. Es ist beispielsweise bekannt, dass mit geringen H₂O₂-Konzentrationen Laborräume, Lagerhallen oder andere Einrichtungen sterilisiert werden, ohne dass auf die H₂O₂-Beständigkeit der begasten Materialien geachtet werden muss. Diese Materialien verbleiben bei der Sterilisation in den Räumen. Die geringe Atmosphäre ist dabei nicht korrosiv und der Sterilisationserfolg wird durch eine lange Behandlungsdauer erzielt.

Bevorzugt werden wenigstens Bestandteil der Umformungseinrichtung uns besonders bevorzugt wird die Umformungseinrichtung permanent, insbesondere auch während der Produktion permanent mit geringen Konzentrationen an Sterilisationsmitteln beaufschlagt und insbesondere begast, wie beispielsweise H₂O₂. Mit einer derartigen Methode müssen keine konstruktiven Änderungen an der Umformungseinrichtung vorgenommen werden, insbesondere da kleine Konzentrationen des Sterilisationsmittels nicht korrosiv wirken.

Bevorzugt ist daher die Beaufschlagungseinrichtung derart gestaltet, dass der Transportraum und/ oder Einrichtungen innerhalb dieses Transportraums mit einer Konzentration eines Sterilisationsmedium beaufschlagt werden oder wird, die geringer ist als die Sterilisation des Sterilisationsmediums in der Sterilisationseinrichtung. Vorteilhaft ist diese Konzentration kleiner als 1.000 ppm, bevorzugt kleiner als 800 ppm, bevorzugt kleiner als 700 ppm, bevorzugt kleiner als 600 ppm und besonders bevorzugt kleiner als 500 ppm. Besonders bevorzugt ist diese Konzentration größer als 30 ppm, bevorzugt größer als 40 ppm und bevorzugt größer als 50 ppm.

Die Vorgehensweise hat auch den Vorteil, dass das Sterilisationsmedium, welches ohnehin gewissermaßen als Abfallprodukt in der Sterilisationseinrichtung abfällt, dazu verwendet werden kann, um die Umformungseinrichtung zu sterilisieren. So kann beispielsweise die Beaufschlagungseinrichtung eine Abluftklappe aufweisen, welche insbesondere zwischen der Sterilisationseinrichtung und der Umformungseinrichtung angeordnet ist. Über diese Abluftklappe kann das Sterilisationsmedium von der Sterilisationseinrichtung zu der Umformungseinrichtung gelangen.

Damit kann bei dieser bevorzugten Ausgestaltung eine Bereitstellung des Sterilisationsmediums, beispielsweise des H₂O₂-Gases, über ein bewusstes Ausströmen des Behandlungsgases aus der Sterilisationseinrichtung erfolgen. Die besagte Abluftklappe zwischen Sterilisationseinrichtung und der Umformungseinrichtung kann dabei derart geregelt werden, dass durch einen leichten Überdruck innerhalb der gesamten Anlage das Sterilisationsmittel, beispielsweise das Sterilisationsgas, in Richtung der Umformungseinrichtung gedrängt bzw. gedrückt wird. Auf diese Weise entsteht auch kein zusätzlicher Verbrauch an H₂O₂, vielmehr wird das schon zur Behandlung der Kunststoffvorformlinge genutzte Gas sekundär auch für eine Oberflächensterilisation der Umformungseinrichtung verwendet und damit recycelt.

Bei einer weiteren vorteilhaften Ausführungsform weist die Vorrichtung bzw. die Beaufschlagungseinrichtung eine Steuerungseinrichtung auf, welche eine Strömung des Sterilisationsmediums von der Sterilisationseinrichtung hin zu der Umformungseinrichtung steuert bzw. regelt. Diese Steuerungseinrichtung kann dabei bevorzugt im Bedarfsfall die Menge an Sterilisationsmedium welches zu der Umformungseinrichtung gelangt steuern bzw. regeln.

Bei einer weiteren bevorzugten Ausführungsform weist daher die Beaufschlagungseinrichtung eine Abluftklappe auf, welche zwischen der Sterilisationseinrichtung und der Umformungseinrichtung angeordnet ist.

Bei einer weiteren vorteilhaften Ausführungsform weist die Vorrichtung eine Fülleinrichtung auf, welche in der Transportrichtung der Kunststoffbehältnisse nach der Umformungseinrichtung angeordnet ist. Diese Fülleinrichtung dient dabei insbesondere zum Befüllen der nunmehr gefertigten Kunststoffbehältnisse mit einer Flüssigkeit und insbesondere mit einem Getränk.

Bei einer weiteren vorteilhaften Ausführungsform weist die Vorrichtung eine Absaugeinrichtung auf, um ein gasförmiges Medium aus dem Transportraum der Umformungseinrichtung abzusaugen. Durch diese Vorgehensweise kann auch erreicht werden, dass kein Sterilisationsmedium zu der Fülleinrichtung gelangt. Weiterhin ist es auch möglich, dass der Umformungseinrichtung gefilterte und/ oder getrocknete Luft zugeführt wird, und dies bevorzugt zusätzlich zu der Zuführung von Sterilisationsmedium erfolgt. Durch die Absaugung eines fließfähigen und insbesondere gasförmigen Mediums aus der Umformungseinrichtung kann auch ein Belüftungskreislauf innerhalb der Umformungseinrichtung erreicht werden.

Vorteilhaft wird im Inneren des Transportraums auch eine Strömung des Sterilisationsmittels und/oder der Luft vorgeschlagen. Bei einer weiteren vorteilhaften Ausführungsform ist auch eine Beaufschlagungseinrichtung vorgesehen, welche die Fülleinrichtung mit einem gasförmigen Medium und insbesondere mit Luft versorgt. Vorteilhaft können dabei diese Belüftungseinrichtungen Elemente, wie etwa Vorfilter, Lufttrockner oder Temperierung aufweisen, sodass es möglich ist, der Umformungseinrichtung und oder der Fülleinrichtung gefilterte und/ oder getrocknete Luft zuzuführen. Bei einer bevorzugten Ausführungsform sind Einrichtungen vorgesehen, welche ein Austreten des Sterilisationsmittels aus der Umformungseinrichtung zu der Fülleinrichtung verhindern. So könnte beispielsweise in einem Bereich des Gehäuses und insbesondere einem Bereich des Gehäuses, in dem die Kunststoffflaschen wieder aus der Umformungseinrichtung austreten, ein umlaufendes und/ oder geschlitztes Rohr oder ein Kanal vorgesehen sein, über welchen die Gasatmosphäre beispielsweise über ein Abluftgebläse abgesaugt wird.

Bei einer weiteren vorteilhaften Ausführungsform weist die Umformungseinrichtung eine Temperaturmesseinrichtung auf. Bevorzugt ist es möglich, dass die Beaufschlagungseinrichtung für das Sterilisationsmedium, beispielsweise eine Abluftklappe, auch in Abhängigkeit der innerhalb der Umformungseinrichtung gemessenen Temperatur gesteuert und/oder geregelt wird. So kann beispielsweise in Abhängigkeit von dieser gemessenen Temperatur eine Konzentration über die Druckdifferenz zwischen der Sterilisationseinrichtung und der Umformungseinrichtung geregelt werden. So wäre es beispielsweise auch möglich, dass während eines Blasformwechsels eine höhere Konzentration an Sterilisationsmedium in der Umformungseinrichtung eingeregelt wird und auf diese Weise auch zusätzlich die Blasformen sterilisiert werden. Daneben wäre es auch möglich, dass insbesondere mit geringen Gaskonzentrationen bzw. Sterilisationsmittelkonzentrationen auch die Blasluftwege sterilisierbar sind.

Bei der hier beschriebenen Weise ist es möglich, ohne großen Konstruktionsaufwand eine Standardumformungseinrichtung bzw. Standardblasformmaschine auf ein hygienisch hohes Niveau zu bringen. Da zur Sterilisation der Umformungseinrichtung das verwendete H₂O₂-Gas bereits vorhanden ist, entstehen auch keine höheren Kosten bzw. Materialverbräuche.

Bei einer weiteren vorteilhaften Ausführungsform weist die Vorrichtung eine Konditioniereinrichtung zum Konditionieren wenigstens eines der Vorrichtung zuzuführenden Mediums auf und insbesondere eine Temperiereinrichtung zum Temperieren wenigstens eines der Vorrichtung zuzuführenden Mediums.

Wie erwähnt, wird eine permanente Begasung mit Sterilisationsmedium und insbesondere verdampftem H₂O₂ für das Blasmodul bzw. die Umformungseinrichtung vorgeschlagen. Da im Blasmodul eine genaue und insbesondere die punktgenaue Einstellung der H₂O₂ Konzentration zum einen wichtig für das Aufrechthalten des keimarmen Zustands ist (es darf keine zu geringe Konzentration vorliegen) und zum anderen für die Standzeit der Anlage (es darf keine zu hohe Konzentration vorliegen, damit Bauteile, wie beispielesweise Buntmetalle nicht korodieren), wird in dieser Ausführungsform vorgschlagen die H₂O₂ Konzentration über eine Konditionierung und insbesondere über eine Taupunktregelung zu gewährleisten. Bevorzugt nimmt daher die Temperiereinrichtung eine Taupunktregelung und insbesondere eine Taupunktregelung des der Umformungseinrichtung zugeführten gasförmigen Mediums vor.

Bei einer besonders bevorzugten Ausführungsform steht die Konditioniereinrichtung wenigstens zeitweise mit der Umformungseinrichtung und insbesondere dem Transportraum der Umformungseinrichtung in Strömungsverbindung. Diese Strömungsverbindung kann dabei insbesondere über Verbindungsleitungen erfolgen.

Bei einer bevorzugten Ausführungsform weist die Konditioniereinrichtung wenigstens eine Wärmetauschereinrichtung auf. Besonders bevorzugt ist diese Wärmetauschereinrichtung steuerbar und/oder regelbar, so dass insbesondere die Temperatur des in die Umformungseinrichtung eintretenden Sterilisationsmediums (wie insbesondere des H₂O₂) geregelt werden kann.

Bei einer weiteren Ausführungsform weist die Konditioniereinrichtung eine Entfeuchtereinrichtung auf. Bevorzugt handelt es sich bei der genannten Wärmetauschereinrichtung um die Entfeuchtereinrichtung.

Bei einer weiteren bevorzugten Ausführungsform weist die Konditioniereinrichtung wenigstens eine Temperaturmesseinrichtung auf, um eine Temperatur zu bestimmen. Dabei kann es sich insbesondere um eine Temperatur in einem Innenbereich der Umformungseinrichtung handeln.

Es wäre jedoch auch (alternativ oder zusätzlich) möglich, dass eine Temperatur des der Umformungseinrichtung zuzuführenden Sterilisationsmediums gemessen wird. So könnte etwa die Temperatur des Sterilisatonsmediums in einer Zuführleitung für das Sterilisationsmedium gemessen werden. Auch könnte die Temperatur im Innenraumbereich der Umformungseinrichtung gemessen werden.

Hierbei wird bevorzugt eine hohe Gaskonzentration, wie zur Packmittelentkeimung bekannt, erzeugt. Dieses Gas wird über einen Entfeuchter auf eine gewünschte Temperatur temperiert und insbesondere abgekühlt, um die überschüssige H₂O₂ Konzentration durch die Sättigung einzustellen.

Bei einer Ausführungsform wird ein Wärmetauscher mit Temperiermedium durchfahren, das H₂O₂ kondensiert bis zur Sättigungsgrenze und kann ausgetragen werden. Die Beaufschlagung des Blasmoduls bzw. der Umformungseinrichtung erfolgt somit weder mit zu niedriger noch zu hoher H₂O₂ Konznentration.

Für diese Vorgehensweise sind mehrere Varianten möglich. So wäre es denkbar, das Sterilisationsmedium wie etwa H₂O₂ direkt aus der zugeführten Behandlungsluft zu entnehmen und über einen Entfeuchter zu konditionieren. Eine Temperaturmessung könnte, wie oben ausgeführt entweder in der Zuleitung oder direkt im Innenraumbereich der Blasmaschine erfolgen.

Bei einer weiteren Variante erfolgt eine Entnahme des H₂O₂ an der Absaugstelle nach dem Behandlungsmodul und die Zuführung (bevorzugt mittels eines Gebläses) über den Entfeuchter und eine Zuführung in das Blasmodul bzw. die Umformungseinrichtung.

Der Vorteil der Temperaturmessung im Innenraum der Blasmaschine besteht darin, dass die Mischtemperatur, welche sich aus der Mischung zwischen Behandlungsluft und zugeführter Luft aus der Lüftungstechnik entsteht, erfasst wird. Ein nachträgliches Abkühlen und damit Kondensieren des Sterilisationsmittel kann so ausgeschlossen werden.

Die vorliegende Erfindung ist weiterhin auf ein Verfahren zum Herstellen von Getränkebehältnissen gerichtet, wobei Kunststoffvorformlinge mittels einer Erwärmungseinrichtung erwärmt werden und nach dieser Erwärmung zu einer Sterilisationseinrichtung transportiert werden und dort mittels dieser Sterilisationseinrichtung sterilisiert werden. Weiterhin werden die Kunststoffvorformlinge von dieser Sterilisationseinrichtung zu einer Umformungseinrichtung transportiert und von dieser Umformungseinrichtung durch Beaufschlagung mit einem fließfähigen Medium zu Kunststoffbehältnissen und insbesondere Kunststoffflaschen expandiert. Dabei werden die Kunststoffvorformlinge und insbesondere Kunststoffflaschen während ihrer Expansion durch einen Transportraum der Umformungseinrichtung transportiert. Erfindungsgemäß wird dieser Transportraum der Umformungseinrichtung und/oder Bestandteile innerhalb dieses Transportraums wenigstens zeitweise mit einem fließfähigen Sterilisationsmedium beaufschlagt.

Bei einem bevorzugten Verfahren strömt wenigstens zeitweise ein Sterilisationsmedium von der Sterilisationseinrichtung zu der Umformungseinrichtung. In diesem Falle erfolgt daher die Beaufschlagung der Umformungseinrichtung durch ein von der Sterilisationseinrichtung stammendes Sterilisationsmedium.

Bei einem weiteren bevorzugten Verfahren wird wenigstens zeitweise das Sterilisationsmedium aus dem Transportraum der Umformungseinrichtung abgesaugt.

Bei einem weiteren bevorzugten Verfahren wird wenigstens zeitweise das fließfähige Medium, insbesondere das Sterilisationsmedium konditioniert und insbesondere temperiert. Besonders bevorzugt erfolgt dieses Konditionieren und insbesondere Temperieren mittels einer Wärmetauschereinrichtung. Besonders bevorzugt handelt es sich bei der Wärmetauschereinrichtung um eine gasbetriebene oder eine flüssigkeitsbetriebene Wärmetauschereinrichtung.

Besonders bevorzugt wird ein Taupunkt des Sterilisationsmediums geregelt. Besonders bevorzugt wird das Sterilisationsgas mittels eines Entfeuchters auf eine vorgegebene Temperatur abgekühlt. Besonders bevorzugt wird sie eine Konzentration und insbesondere eine H₂O₂ Konzentration durch die Sättigung eingestellt.

Besonders bevorzugt wird eine Temperatur des Sterilisationsmediums und/oder des Innenraums der Umformungseinrichtung wenigstens zeitweise gemessen. Bei einem weiteren bevorzugten Verfahren wird die Konditioniereinrichtung auf Basis dieser Temperaturmessungen gesteuert.

Weitere Vorteile und Ausführungsformen ergeben sich aus den beigefügten Zeichnungen, dabei zeigt:
- Fig. 1: Eine schematische Darstellung einer erfindungsgemäßen Anlage zum Herstellen von Getränkebehältnissen;
- Fig. 2: eine Darstellung einer Anlage mit Konditioniereinrichtung in einer ersten Ausführungsform;
- Fig. 3: eine Darstellung einer Anlage mit Konditioniereinrichtung in einer zweiten Ausführungsform; und
- Fig. 4: eine Darstellung einer Anlage mit Konditioniereinrichtung in einer dritten Ausführungsform.

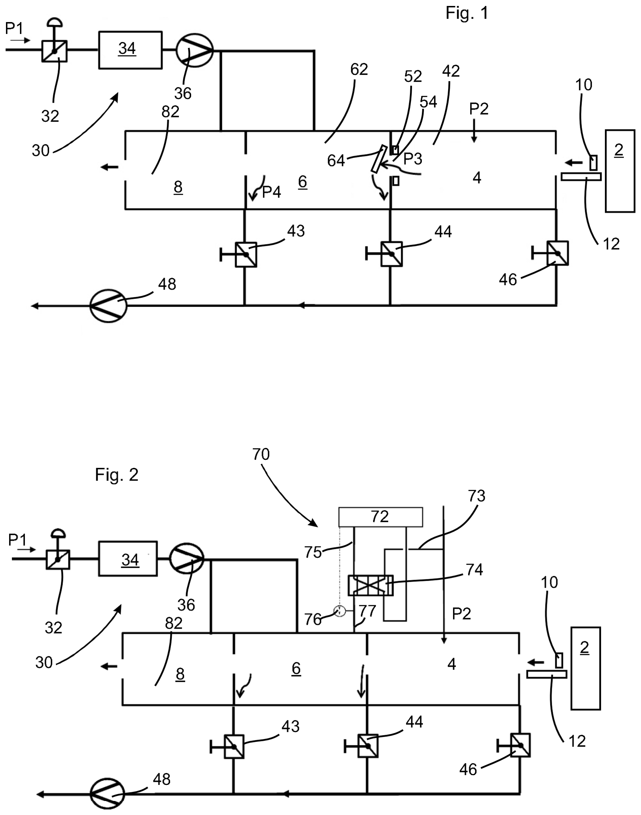

Figur 1 zeigt eine schematische Darstellung einer erfindungsgemäßen Vorrichtung 1 zum Herstellen von Getränkebehältnissen. Dabei bezieht sich das Bezugszeichen 2 auf eine Erwärmungseinrichtung, welche dazu geeignet und bestimmt ist, Kunststoffvorformlinge 10 zu erwärmen. Die Kunststoffvorformlinge 10 werden dann erwärmt mit einer Transporteinrichtung 12 zunächst zu einer Sterilisationseinrichtung 4 transportiert. Es wird darauf hingewiesen, dass sich die Transporteinrichtung auch im Wesentlichen durch die gesamte Anlage erstreckt. Auch kann diese Transporteinrichtung mehrere Transporteinheiten, wie beispielsweise Transportsterne, Transportbänder und dergleichen aufweisen, welche sich durch die gesamte Anlage erstrecken. Auch bei dem oben erwähnten und in der Figur nicht gezeigten Blasrad handelt es sich um eine solche Transporteinrichtung.

Innerhalb der Sterilisationseinrichtung werden die Kunststoffvorformlinge mit einem Sterilisationsmedium, beispielsweise einem H₂O₂-Gas beaufschlagt. Das Bezugszeichen 42 kennzeichnet einen Transportraum, innerhalb dessen die Kunststoffvorformlinge während ihrer Sterilisation transportiert werden. Dieser Transportraum 42 wird mit einer Atmosphäre des Sterilisationsmediums beaufschlagt, wie durch den Pfeil P2 angedeutet. Das Bezugszeichen 6 kennzeichnet eine Umformungseinrichtung, innerhalb derer die Kunststoffvorformlinge durch Expansion bzw. Beaufschlagung mit Blasluft zu den Kunststoffflaschen umgeformt werden.

Diese Umformungseinrichtung weist dabei ebenfalls einen Transportraum 62 auf, innerhalb dessen ebenfalls eine Transporteinrichtung angeordnet ist, welche die Kunststoffvorformlinge transportiert. Diese Transporteinrichtung ist dabei insbesondere als (nicht gezeigtes) Blasrad ausgebildet, an dem eine Vielzahl von Umformungsstationen angeordnet ist. Diese Umformungsstationen dienen dabei jeweils zum Umformen der Kunststoffvorformlinge zu Kunststoffbehältnissen. Das Blasrad dreht sich zu diesem Zweck und damit werden die Kunststoffvorformlinge in einem Rundläuferbetrieb expandiert. Die einzelnen Umformungsstationen weisen dabei wie oben erwähnt bevorzugt Blasformträger auf, an denen zumindest mittelbar Blasformteile angeordnet sind.

Das Bezugszeichen P3 kennzeichnet einen Übergang des Sterilisationsmediums von dem Transportraum 42 der Sterilisationseinrichtung 4 in den Transportraum 62 der Umformungseinrichtung 6. Das Bezugszeichen 64 kennzeichnet zu diesem Zweck eine Belüftungsklappe, die geöffnet oder mehr oder weniger geschlossen werden kann, um auf diese Weise den Durchgang des Sterilisationsmediums von der Sterilisationseinrichtung 4 zu der Umformungseinrichtung 6 kontrollieren bzw. regeln zu können.

Das Bezugszeichen 8 kennzeichnet eine Fülleinrichtung, welche die nunmehr von der Umformungseinrichtung 6 ausgeformten Behältnisse mit einem Füllgut und insbesondere einem Getränk befüllt. Die Fülleinrichtung 8 weist zu diesem Zweck ebenfalls einen Transportraum 62 auf, innerhalb dessen die Kunststoffbehältnisse während der Befüllung transportiert werden. Vorteilhaft kann auch die Fülleinrichtung ein Füllrad aufweisen, an dem eine Vielzahl von Füllstationen angeordnet ist, wobei jede dieser Füllstationen dazu geeignet und bestimmt ist, die Kunststoffflaschen mit einem Getränk zu befüllen.

Dabei herrscht, wie oben erwähnt in der Sterilisationseinrichtung bzw. dem Transportraum 42 ein höheres Niveau bzw. eine höhere Konzentration an Sterilisationsmittel als in dem Transportraum 62 der Umformungseinrichtung 6. Vorzugsweise soll auch in dem Transportraum 82 der Fülleinrichtung keinerlei Sterilisationsmittelatmosphäre mehr herrschen bzw. dort die Konzentration im Wesentlichen 0 sein.

Die Vorrichtung weist weiterhin eine Belüftungseinrichtung 30 auf, welche zum Belüften insbesondere der Umformungseinrichtung 6 und der Fülleinrichtung 8 dient. Die Belüftungseinrichtung 30 weist dabei zunächst eine Ventileinrichtung 32 auf, der Zuluft (Pfeil P1) zugeführt wird. Das Bezugszeichen 34 kennzeichnet eine Luftkonditionierungseinheit, insbesondere eine Einheit, welche einen Vorfilter, einen Lufttrockner und eine Temperierungseinheit aufweisen kann. Das so vorkonditionierte Gas bzw. die Luft wird über ein Gebläse 36 sowohl der Fülleinrichtung 8 als auch der Umformungseinrichtung 6 zugeführt.

Die Bezugszeichen 43, 44 und 46 kennzeichnen Ventileinrichtungen, welche eine Absaugung von Medien aus den einzelnen Stationen, das heißt der Sterilisationseinrichtung 4, der Umformungseinrichtung 6 und der Fülleinrichtung 8 regeln können. Das Bezugszeichen 48 schließlich kennzeichnet eine Absaugeinrichtung wie ein Gebläse bzw. Pumpe, welche dazu dient, um die Luft abfördern zu können. Im Rahmen dieser Steuerung dieser einzelnen Ventile 42, 44 und 46 kann insbesondere gesteuert bzw. geregelt werden, in welchem Maß bzw. welchem Umfang Luft bzw. auch Sterilisationsmittel enthaltende Luft aus den einzelnen Einrichtungen abführbar ist.

Das Bezugszeichen 52 kennzeichnet eine spezielle Absaugeinrichtung, welche an einem Ausgang 54 der Umformungseinrichtung angeordnet ist und welche insbesondere dazu dient, um in diesem Bereich Sterilisationsmedium abzusaugen. Auf diese Weise kann verhindert werden, dass in die Fülleinrichtung Sterilisationsmedium bzw. Reste von Sterilisationsmedium gelangen.

Dabei ist wie oben erwähnt diese Absaugeinrichtung als umlaufendes Rohr oder Kanal ausgebildet, welches bevorzugt geschlitzt ist und welches besonders bevorzugt die Gasatmosphäre über das ohnehin vorhandene Abluftgebläse 48 aus der Umformungseinrichtung 6 abführen kann.

Fig. 2 zeigt eine weitere Ausführungsform einer erfindungsgemäßen Anlage. Bei dieser Ausführungsform weist die Anlage eine in ihrer Gesamtheit mit 70 bezeichnete Konditionier- und insbesondere Temperiereinrichtung für das der Umformungseinrichtung 6 zuzuführende Sterilisationsmedium auf.

Diese Temperiereinrichtung weist eine Wärmetauschereinrichtung 74 auf, der über eine Zuführleitung 73 das Sterilisationsmedium zugeführt wird. Dabei kann diese Zuführleitung 73 von einer Hauptleitung abgezweigt sein, aber auch direkt in diese Wärmetauschereinrichtung münden.

Das Bezugszeichen 72 kennzeichnet eine Steuerungseinrichtung für die Wärmetauschereinrichtung 74. Diese steuert insbesondere die Temperatur eines Temperiermittels, welches über eine Leitung 75 der Wärmetauschereinrichtung 74 zugeführt wird.

Das Bezugszeichen 76 kennzeichnet eine Temperaturmesseinrichtung, welche eine Temperatur des Sterilisationsmittels in der Leitung 77 bestimmt.

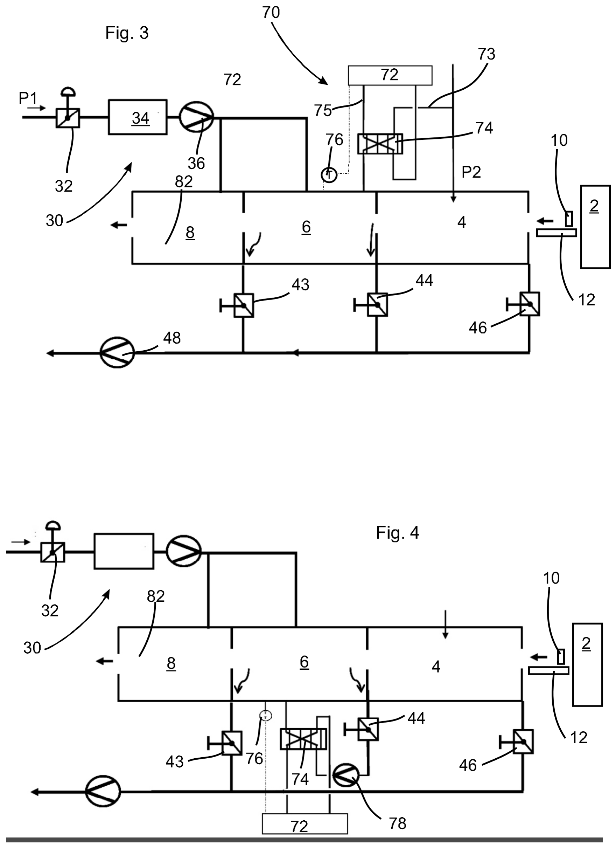

Fig. 3 zeigt eine weitere Ausführungsform der in Fig. 2 gezeigten Anlage, welche sich insbesondere durch die Anordnung der Temperaturmesseinrichtung unterscheidet. Bei dieser Ausführungsform misst die Temperaturmesseinrichtung 76 die Temperatur direkt in dem Raum 62 der Umformungseinrichtung. Bei beiden Ausgestaltungen wird die Konditioniereinrichtung auch unter Berücksichtigung der gemessenen Temperatur gesteuert.

Bei einer weiteren Ausgestaltung wäre es auch möglich, dass zwei oder mehrere Temperaturmesseinrichtungen vorgesehen, sind, etwa an den Position wie durch Fig. 2 und Fig. 3 gezeigt.

Fig. 4 zeigt eine weitere Ausführungsform der erfindungsgemäßen Anlage mit Konditioniereinrichtung. Bei dieser Ausgestaltung wird Sterilisationsmedium von dem Behandlungsmodul abgenommen und über eine Gebläseeinrichtung 78 der Umformungseinrichtung 6 zugeführt. Die Wärmetauschereinrichtung arbeitet hier in der gleichen Weise wie oben beschrieben.

Die Anmelderin behält sich vor sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können. Es wird weiterhin darauf hingewiesen, dass Merkmale, welche hier unter Bezugnahme auf die Vorrichtung beschrieben werden auch im Hinblick auf das ebenfalls beschriebene Verfahren verwendet werden.

### Bezugszeichenliste

- 2: Erwärmungseinrichtung
- 4: Sterilisationseinrichtung
- 6: Umformungseinrichtung
- 8: Fülleinrichtung
- 10: Kunststoffvorformlinge
- 12: Transporteinrichtung
- 30: Belüftungseinrichtung
- 32: Ventileinrichtung
- 34: Luftkonditionierungseinheit
- 36: Gebläse
- 42: Transportraum
- 43: Ventileinrichtung
- 44: Ventileinrichtung
- 46: Ventileinrichtung
- 48: Absaugeinrichtung, Abluftgebläse
- 52: Absaugeinrichtung
- 54: Ausgang der Umformungseinrichtung
- 62: Transportraum
- 64: Belüftungsklappe
- 70: Konditioniereinrichtung
- 72: Steuerungseinrichtung
- 73: Leitung
- 74: Wärmetauschereinrichtung
- 75: Leitung
- 76: Temperaturmesseinrichtung78 Pumpeneinrichtung, Gebläse
- 82: Transportraum
- P1: Zuluft
- P2: Atmosphäre des Sterilisationsmediums
- P3: Übergang des Sterilisationsmediums

## Patentansprüche

1. Vorrichtung zum Herstellen von Behältnissen und insbesondere von Getränkebehältnissen mit einer Erwärmungseinrichtung (2), welche dazu geeignet und bestimmt ist, Kunststoffvorformlinge zu erwärmen, mit wenigstens einer ersten Transporteinrichtung (12), welche dieses Kunststoffvorformlinge (10) entlang eines vorgegebenen Transportpfads transportiert, mit einer Sterilisationseinrichtung (4), welche dazu geeignet und bestimmt ist, die Kunststoffvorformlinge (10) zu sterilisieren und mit einer in einer Transportrichtung der Kunststoffvorformlinge (10) nach der Sterilisationseinrichtung (4) angeordneten Umformungseinrichtung (6), welche dazu geeignet und bestimmt ist, die Kunststoffvorformlinge zu Kunststoffbehältnissen durch Beaufschlagung mit einem fließfähigen Medium umzuformen, wobei diese Umformungseinrichtung (6) einen Transportraum (62) aufweist, innerhalb dessen die Kunststoffvorformlinge (10) transportiert werden
**dadurch gekennzeichnet, dass**
die Vorrichtung (1) eine Beaufschlagungseinrichtung (64) aufweist, welche diesen Transportraum (62) mit einem fließfähigen Sterilisationsmedium beaufschlagt.

2. Vorrichtung (1) nach Anspruch 1,
**dadurch gekennzeichnet, dass**
die Sterilisationseinrichtung (4) wenigstens eine Beaufschlagungseinrichtung (42) aufweist, um die Kunststoffvorformlinge mit dem fließfähigen Sterilisationsmedium zu beaufschlagen.

3. Vorrichtung (1) nach Anspruch 1,
**dadurch gekennzeichnet, dass**
die Sterilisationseinrichtung (4) mit der Umformungseinrichtung (6) derart in Strömungsverbindung steht, dass das Sterilisationsmedium von der Sterilisationseinrichtung (4) zu der Umformungseinrichtung (6) gelangen kann.

4. Vorrichtung (1) nach wenigstens einem der vorangegangenen Ansprüche,
**dadurch gekennzeichnet, dass**
die Beaufschlagungseinrichtung (64) derart gestaltet ist, dass der Transportraum (62) mit einer Konzentration an Sterilisationsmedium beaufschlagt wird, die geringer ist als die Konzentration dieses Sterilisationsmediums in der Sterilisationseinrichtung (4).

5. Vorrichtung (1) nach wenigstens einem der vorangegangenen Ansprüche,
**dadurch gekennzeichnet, dass**
die Beaufschlagungseinrichtung eine Abluftklappe aufweist, welche zwischen der Sterilisationseinrichtung (4) und der Umformungseinrichtung (6) angeordnet ist.

6. Vorrichtung (1) nach wenigstens einem der vorangegangenen Ansprüche,
**dadurch gekennzeichnet, dass**
die Vorrichtung eine Fülleinrichtung (8) aufweist, welche in der Transportrichtung der Kunststoffbehältnisse nach der Umformungseinrichtung (6) angeordnet ist.

7. Vorrichtung (1) nach wenigstens einem der vorangegangenen Ansprüche,
**dadurch gekennzeichnet, dass**
die Vorrichtung (1) eine Absaugeinrichtung aufweist, um ein gasförmiges Medium aus dem Transportraum (62) der Umformungseinrichtung abzusaugen.

8. Vorrichtung (1) nach wenigstens einem der vorangegangenen Ansprüche,
**dadurch gekennzeichnet, dass**
die Vorrichtung (1) eine Gasbeaufschlagungseinrichtung aufweist, welche die Umformungseinrichtung (6) mit einem Gas und insbesondere mit Luft beaufschlagt.

9. Vorrichtung (1) nach wenigstens einem der vorangegangenen Ansprüche,
**dadurch gekennzeichnet, dass**
die Vorrichtung eine Konditioniereinrichtung (70) zum Konditionieren wenigstens eines der Vorrichtung zuzuführenden Mediums aufweist und insbesondere eine Temperiereinrichtung (70) zum Temperieren wenigstens eines der Vorrichtung zuzuführenden Mediums, wobei bevorzugt die Konditioniereinrichtung wenigstens zeitweise mit dem Transportraum der Beaufschlagungseinrichtung in Strömungsverbindung steht.

10. Vorrichtung (1) nach Anspruch 9,
**dadurch gekennzeichnet, dass**
die Konditioniereinrichtung (70) wenigstens eine Wärmetauschereinrichtung (72) aufweist.

11. Vorrichtung (1) nach Anspruch 9,
**dadurch gekennzeichnet, dass**
die Konditioniereinrichtung wenigstens eine Temperaturmesseinrichtung (76), insbesondere zur Messung einer Temperatur des Sterilisationsmediums und/oder einer Temperatur des Transportraums (62) aufweist.

12. Vorrichtung (1) nach wenigstens einem der vorangegangenen Ansprüche,
**dadurch gekennzeichnet, dass**
die Umformungseinrichtung (6) keinen Reinraum aufweist und nicht aseptisch ausgeführt ist.

13. Vorrichtung (1) nach wenigstens einem der vorangegangenen Ansprüche,
**dadurch gekennzeichnet, dass**
es sich bei der Erwärmungseinrichtung (2), welche der Umformungseinrichtung (6) vorgeschaltet ist, um einen Mikrowellenofen handelt, der die Kunststoffvorformlinge (10) durch Beaufschlagung mit Mikrowellen erwärmt oder, es sich bei der Erwärmungseinrichtung (2) um eine Spritzgießmaschine handelt, so dass die Kunststoffvorformlinge (10) direkt aus einer Spritzgießmaschine stammen und eine hohe Eigentemperatur aufweisen.

14. Vorrichtung (1) nach wenigstens einem der vorangegangenen Ansprüche,
**dadurch gekennzeichnet, dass**
ein Taupunkt des Sterilisationsmediums geregelt wird.

15. Verfahren zum Herstellen von Getränkebehältnissen, wobei Kunststoffvorformlinge mittels einer Erwärmungseinrichtung (2) erwärmt werden und nach dieser Erwärmung zu einer Sterilisationseinrichtung (4) mittels einer Sterilisationseinrichtung (4) sterilisiert werden und von dieser Sterilisationseinrichtung (4) zu einer Umformungseinrichtung (6) transportiert werden und von dieser Umformungseinrichtung (6) durch Beaufschlagung mit einem fließfähigen Medium zu Kunststoffbehältnissen und insbesondere Kunststoffflaschen expandiert werden, wobei die Kunststoffvorformlinge während ihrer Expansion durch einen Transportraum (62) der Umformungseinrichtung (6) transportiert werden,
**dadurch gekennzeichnet, dass**
dieser Transportraum (62) der Umformungseinrichtung (6) wenigstens zeitweise mit einem fließfähigen Sterilisationsmedium beaufschlagt wird.

16. Verfahren nach dem vorangegangenen Anspruch,
**dadurch gekennzeichnet, dass**
wenigstens zeitweise das Sterilisationsmedium von der Sterilisationseinrichtung (4) zu der Umformungseinrichtung (6) strömt und/oder wenigstens zeitweise das Sterilisationsmedium aus dem Transportraum (62) der Umformungseinrichtung (6) abgesaugt wird.

17. Verfahren nach wenigstens einem der vorangegangenen Ansprüche,
**dadurch gekennzeichnet, dass**
wenigstens Bestandteile der Umformungseinrichtung und besonders bevorzugt die Umformungseinrichtung permanent mit geringen Konzentrationen an Sterilisationsmitteln begast werden.

18. Verfahren nach wenigstens einem der vorangegangenen Ansprüche,
**dadurch gekennzeichnet, dass**
wenigstens zeitweise das fließfähige Medium konditioniert und insbesondere temperiert wird.
